(19) Europäisches Patentamt
European Patent Office
Office européen des brevets

(11) Numéro de publication : **0 347 340 B1**

(12) # FASCICULE DE BREVET EUROPEEN

(45) Date de publication du fascicule du brevet : **08.04.92 Bulletin 92/15**

(51) Int. Cl.[5] : **C07C 69/593,** C07C 67/36

(21) Numéro de dépôt : **89420199.5**

(22) Date de dépôt : **08.06.89**

(54) **Procédé de préparation de diesters de l'acide hexènedioique.**

(30) Priorité : **13.06.88 FR 8808197**

(43) Date de publication de la demande : **20.12.89 Bulletin 89/51**

(45) Mention de la délivrance du brevet : **08.04.92 Bulletin 92/15**

(84) Etats contractants désignés : **AT BE CH DE ES FR GB GR IT LI LU NL SE**

(56) Documents cités :
**US-A- 3 367 961**
**US-A- 4 060 547**
**US-A- 4 611 082**

(73) Titulaire : **RHONE-POULENC CHIMIE**
**25, quai Paul Doumer**
**F-92408 Courbevoie Cédex (FR)**

(72) Inventeur : **Deweerdt, Hélène**
**Résidence de l'Ile Chemin de la Loge**
**F-31400 Toulouse (FR)**
Inventeur : **Jenck, Jean**
**1, Impasse du Rotagnier**
**F-69680 Chassieu (FR)**
Inventeur : **Kalck, Philippe**
**La Pradine Tolozane No 4**
**Auzeville F-31320 Castanet Tolosan (FR)**

(74) Mandataire : **Varnière-Grange, Monique et al**
**RHONE-POULENC CHIMIE Service Brevets**
**Chimie Centre de Recherches des Carrières**
**B.P. 62**
**F-69192 Saint-Fons Cédex (FR)**

EP 0 347 340 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé de préparation de diesters de l'acide hexènedioïque-1,6. Ces diesters peuvent être hydrogénés en diesters de l'acide adipique correspondants ou adipates qui peuvent alors être hydrolysés pour former l'acide adipique. L'acide adipique, l'une des matières premières du nylon 66 est produit avec de forts tonnages et de ce seul fait, toute nouvelle voie d'accès à ce diacide et/ou à ses dérivés présente un intérêt de principe immédiatement perceptible.

La présente invention a plus spécifiquement pour objet un procédé de préparation de diesters de l'acide hexène-3 dioïque-1,6 par réaction du monoxyde de carbone et d'un alcool sur au moins un butène disubstitué par des groupes acyloxy en présence d'un catalyseur à base de palladium.

La préparation d'un monoester d'un alcool et d'un acide buténoïque par réaction du monoxyde de carbone et d'un alcool sur un monoester d'un alcool allylique en présence d'un catalyseur à base de palladium est décrite dans le brevet américain n° 3,367,961. Ainsi il est possible d'obtenir du vinylacétate d'éthyle à partir d'acétate d'allyle, de monoxyde de carbone et d'éthanol en présence de chlorure de palladium et de palladium déposé sur charbon.

Il est également indiqué dans le brevet américain n° 4,611,082 que la carbonylation d'une solution du diacétoxy-1,4 butène-2 dans un solvant aprotique, polaire et non basique choisi dans le groupe constitué par les nitriles, le bis(méthoxy-2) butène-2, le bis(méthoxy-2 éthyl)éther et le chlorure de méthylène à 80 - 140°C en présence d'un halogénure d'un métal de transition n'est pratiquement pas observée et qu'en présence d'un alcool les vitesses augmentent et sont comparables à celles constatées pour la carbonylation de butène-2 diol-1,4. A propos de ce dernier substrat mentionné il est également indiqué qu'il ne permet pas d'atteindre dans les conditions précitées des rendements satisfaisants en produits linéaires de carbonylation et, dans ce contexte, préférence est donnée aux substrats substitués en position 1,4 par des groupements alcoxy.

Or le diacétoxy-1,4 butène-2 est aisément accessible par acétoxylation du butadiène. Il serait donc hautement souhaitable de pouvoir disposer d'un procédé permettant d'obtenir avec une efficacité élevée les diesters de l'acide hexène-3 dioïque-1,6 à partir du diacétoxy-1,4 butène-2, par exemple, et plus généralement de butènes disubstitués par des groupes acyloxy.

La présente invention a donc pour objet un procédé de préparation de diesters de l'acide hexène-3 dioïque par réaction du monoxyde de carbone et d'un alcool sur au moins un butène disubstitué par des groupes acyloxy en présence d'un catalyseur à base de palladium caractérisé en ce que la réaction est conduite également en présence d'un halogénure d'onium quaternaire d'un élément du groupe VB choisi parmi l'azote et le phosphore, ledit élément étant tétracoordonné à des atomes de carbone, l'azote pouvant être coordonné à deux atomes de phosphore pentavalents, l'anion halogénure étant choisi parmi le chlorure et le bromure.

Il a en effet été trouvé de manière tout à fait surprenante qu'un tel procédé permet de réaliser la dicarbonylation dans des conditions de pression et de température acceptables à l'échelle industrielle, avec une sélectivité appréciable en produit dicarbonylé linéaire, les proportions de produit monocarbonylé et de produits dicarbonylés branchés étant minimes.

Le procédé en cause peut être représenté par le schéma réactionnel suivant, lorsqu'on part d'un butène-2 disubstitué en 1,4 par des groupes acyloxy,

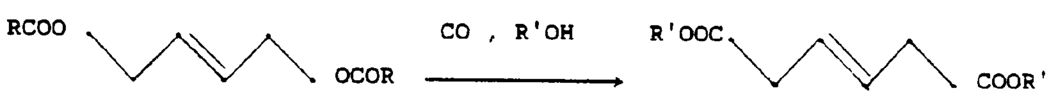


dans lequel :

– R représente :

. un radical alkyle linéaire ou ramifié, contenant de 1 à 12 atomes de carbone, éventuellement substitué par un groupe phényle ;

ou

. un radical aryle contenant de 6 à 10 atomes de carbone, éventuellement substitué par un ou deux radicaux alkyles contenant de 1 à 4 atomes de carbone ;

et peut comporter de 1 à 3 substituants choisis parmi les atomes de fluor et de chlore et les groupements dialkylamino et N,N-dialkylamido dont les radicaux alkyles renferment au plus 4 atomes de carbone.

– R', identique à ou différent de R, représente :

. un radical alkyle linéaire ou ramifié contenant de 1 à 12 atomes de carbone, éventuellement substitué par un groupe phényle ;

ou

. un radical aryle contenant de 6 à 10 atomes de carbone, éventuellement substitué par un ou deux radicaux alkyles contenant de 1 à 4 atomes de carbone ;

et peut comporter de 1 à 3 substituants choisis parmi les atomes de fluor, de brome et de chlore.

Le procédé, selon la présente invention, requiert la mise en oeuvre d'au moins un butène disubstitué par des groupes acyloxy. Par groupe acyloxy, on entend des groupes de formule RCOO- dans laquelle R a la signification donnée précédemment ; par butènes disubstitués on entend les composés du butène-2 substitués en position 1 et 4 et les composés du butène-1 substitués en positions 3 et 4. Bien entendu des mélanges de butène-2 disubstitués par des groupes acyloxy de nature distincte, des mélanges de butène-1 disubstitués par des groupes acyloxy de nature distincte ou des mélanges de butène-2 et de butène-1 disubstitués peuvent être mis en oeuvre dans le cadre du présent procédé.

Il a en effet été constaté par la Demanderesse que la sélectivité en diester linéaire est sensiblement la même que l'on parte d'un butène-2 disubstitué par des groupes acyloxy en position 1,4 ou d'un butène-1 disubstitué par des groupes acyloxy en positions 3 et 4.

A titre d'exemples de butènes disubstitués par des groupes acyloxy on peut citer : les diacétoxybutènes, les dipropionyloxybutènes, les dibutyryloxybutènes et les dibenzoyloxybutènes.

Le diacétoxy-1,4 butène-2, le diacétoxy-3,4-butène-1 et leurs mélanges conviennent plus particulièrement bien à la mise en oeuvre de la présente invention.

Le procédé selon la présente invention requiert également la mise en oeuvre d'un alcool de formule R'OH dans laquelle R' a la signification donnée précédemment.

A titre d'exemple d'alcool convenant à la mise en oeuvre du procédé on peut citer :

le méthanol, l'éthanol, l'isopropanol, le n-propanol, le tertiobutanol, le n-hexanol, l'éthyl-2 hexanol, le nonanol-1, le dodécanol, le phényléthanol, le phénol et le trifluoroéthanol.

On utilise avantageusement un alcanol dont le radical R' comporte au maximum 12 atomes de carbone et, de préférence, 4 atomes de carbone.

La quantité d'alcanol à mettre en oeuvre dans le cadre du présent procédé n'est pas critique et peut varier dans de larges limites.

Pour une bonne mise en oeuvre de la réaction le rapport molaire de l'alcool au butène disubstitué sera compris entre 1 et 100 et, de préférence entre 1 et 50.

Le procédé selon la présente invention est conduit en présence d'un catalyseur à base de palladium.

Bien que la nature précise de l'espèce (ou des espèces) catalytiquement active(s) dans la réaction en cause ne soit pas totalement élucidée, la Demanderesse a constaté que divers composés du palladium et le palladium métallique sont susceptibles d'être des précurseurs utiles dans la mise en oeuvre du présent procédé.

Parmi les sources de palladium pouvant être mises en oeuvre pour réaliser le procédé faisant l'objet de l'invention, on peut citer :

. le palladium métallique déposé le cas échéant sur un support, tel que le charbon, l'alumine, ou la silice,

. $PdCl_2$, $Pd(OAc)_2$

. les sels ou les complexes $\pi$-allyliques de palladium, dont l'anion coordonné au cation Pd est choisi parmi les anions suivants : carboxylates tels que formiate, acétate, propionate, benzoate ; acétylacétonate, halogénures tels que $Cl^-$ et $Br^-$ et de préférence $Cl^-$ ;

La quantité précise de catalyseur à mettre en oeuvre, qui peut varier dans de larges limites, dépendra avant tout d'un compromis entre l'efficacité souhaitée et la dépense en catalyseur et des autres conditions choisies par la réaction. En général de bons résultats sont obtenus avec un rapport molaire butène substitué/Palladium compris entre 10 et 50. Un rapport de l'ordre de 200 peut être mis en oeuvre, la réaction étant plus lente et un rapport de l'ordre de 2 ou moins ne présentant d'obstacles que de nature économique.

L'une des caractéristiques essentielles du présent procédé réside dans le fait que la réaction est conduite également en présence d'un chlorure (ou d'un bromure) d'onium quaternaire d'un élément du groupe VB choisi parmi l'azote et le phosphore, ledit élément étant tétracoordonné à des atomes de carbone, l'azote pouvant en outre être coordonné à deux atomes de phosphore pentavalents.

Par cation onium quaternaire dont l'élément du groupe VB est tétracoordonné à des atomes de carbone, on entend des cations formés à partir d'azote ou de phosphore et de quatre groupements hydrocarbonés monovalents, identiques ou différents, dont la valence libre est portée par un atome de carbone, chaque groupement étant relié à l'élément précité par ladite valence libre, deux quelconques de ces groupements pouvant par ailleurs former ensemble un radical divalent.

Pour une bonne réalisation du procédé de l'invention, le sel d'onium quaternaire présente un cation onium quaternaire répondant à l'une des formules I à IV ci-après :

I)

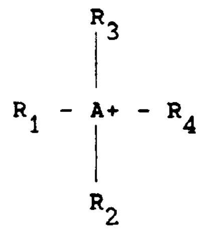

II)

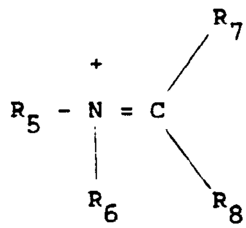

III)

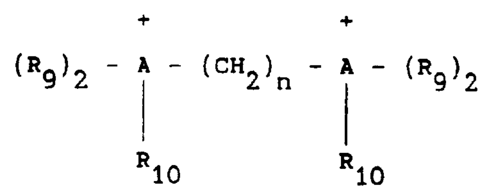

IV)

$$(R_{11})_3 - P = N^+ = P - (R_{11})_3$$

– A représente de l'azote ou du phosphore

– $R_1$, $R_2$,$R_3$, $R_4$ sont identiques ou différents et réprésentent :

. un radical alkyle linéaire ou ramifié contenant de 1 à 16 atomes de carbone, éventuellement substitué par un groupe phényle, hydroxy, halogéno, nitro, alcoxy ou alcoxycarbonyle ;

. un radical alcényle linéaire ou ramifié contenant de 2 à 12 atomes de carbone, de préférence de 4 à 8 atomes de carbone,

. un radical aryle contenant de 6 à 10 atomes de carbone, éventuellement substitué par un ou des radicaux alkyles contenant de 1 à 4 atomes de carbone, alcoxy, alcoxycarbonyle ou halogéno ;

. deux desdits radicaux $R_1$ à $R_4$ pouvant former ensemble un radical alkylène, alcénylène ou alcadiènylène, linéaire ou ramifié et contenant de 3 à 6 atomes de carbone ;

– $R_5$, $R_6$, $R_7$, $R_8$ sont identiques ou différents et représentent :

. un radical alkyle linéaire ou ramifié contenant de 1 à 4 atomes de carbone ;

. les radicaux $R_7$ et $R_8$ pouvant former ensemble un radical alkylène, contenant de 3 à 6 atomes de carbone ;

. les radicaux $R_8$ et $R_7$ ou $R_8$ et $R_8$ pouvant former ensemble un radical alkylène, alcénylène ou alcadiènylène, contenant 4 atomes de carbone et constituant avec N un hétérocycle azoté ;

– $R_9$ représente un radical alkyle linéaire ou ramifié contenant de 1 à 4 atomes de carbone ou un radical phényle ;

– $R_{10}$ représente :

. un radical alkyle linéaire ou ramifié contenant de 1 à 4 atomes de carbone, semblable ou différent de $R_9$ .

. un radical alcényle linéaire ou ramifié, contenant de 2 à 12 atomes de carbone, de préférence de 4 à 8 atomes de carbone ;

– n représente un nombre entier supérieur ou égal à 1 et inférieur ou égal à 10 et de préférence inférieur ou égal à 6 ;

– $R_{11}$ représente un radical aryle contenant de 6 à 10 atomes de carbone, éventuellement substitué par un ou des groupes alkyle contenant de 1 à 4 atomes de carbone, alcoxy, alcoxycarbonyle ou halgéno.

A titre d'exemple de cations onium quaternaire répondant à la formule I on peut citer les cations :

. tétraméthylammonium,
. triéthylméthylammonium
. tributylméthylammonium
. triméthyl(n-propyl)ammonium
. tétraéthylammonium
. tétrabutylammonium
. dodécyltriméthylammonium
. méthyltrioctylammonium
. heptyltributylammonium
. tétrapropylammonium
. tétrapentylammonium
. tétrahexylammonium
. tétraheptylammonium
. tétraoctylammmonium
. tétradécylammonium
. butyltripropylammonium
. méthyltributylammonium
. pentyltributylammonium
. méthyldiéthylpropylammonium
. éthyldiméthylpropylammonium
. tétradodécylammonium
. tétraoctadécylammonium
. hexadécyltriméthylammonium
. benzyltriméthylammonium
. benzyldiméthylpropylammonium
. benzyldiméthyloctylammonium
. benzyltributylammonium
. benzyltriéthylammonium
. phényltriméthylammonium
. benzyldiméthyltétradécylammonium
. benzyldiméthylhexadécylammonium
. diméthyldiphénylammonium
. méthyltriphénylammonium
. butène-2 yltriéthylammonium
. N,N-diméthyl-tétraméthylènammonium
. N,N-diéthyl-tétraméthylènammonium
. tétraméthylphosphonium
. tétrabutylphosphonium
. éthyltriméthylphosphonium
. triméthylpentylphosphonium
. octyltriméthylphosponium
. dodécyltriméthylphosphonium
. triméthylphénylphosphonium
. diéthyldiméthylphosphonium
. dicyclohexyldiméthylphosphonium
. diméthyldiphénylphosponium
. cyclohexyltriméthylphosphonium
. triéthylméthylphosphonium
. méthyl-tri(isopropyl)phosphonium
. méthyl-tri(n-propyl)phosphonium
. méthyl-tri(n-butyl)phosphonium
. méthyl-tri(méthyl-2 propyl)phosphonium
. méthyltricyclohexylphosphonium
. méthyltriphénylphosphonium
. méthyltribenzylphosphonium
. méthyl-tri(méthyl-4 phényl)phosphonium
. méthyltrixylylphosphonium

. diéthylméthylphénylphosphonium
. dibenzylméthylphénylphosphonium
. éthyltriphénylphosphonium
. tétraéthylphosphonium
. éthyl-tri(n-propyl)phosphonium
. triéthylpentylphosphonium
. hexadécyltributylphosphonium
. éthyltriphénylphosphonium
. n-butyl-tri(n-propyl)phosphonium
. butyltriphénylphosphonium
. benzyltriphénylphosphonium
. (β-phényléthyl)diméthylphénylphosphonium
. tétraphénylphosphonium
. triphényl(méthyl-4 phényl)phosphonium
. tétrakis(hydroxyméthyl)phosphonium
. tétrakis(hydroxy-2 éthyl)phosphonium

Parmi les cations répondant à la formule II, on peut citer les cations :
. N-méthylpyridinium
. N-éthylpyridinium
. N-hexadécylpyridinium
. N-méthylpicolinium

Parmi les cations répondant à la formule III, on peut citer les cations :
. bis(triméthylammonium)-1,2 éthane
. bis(triméthylammonium)-1,3 propane
. bis(triméthylammonium)-1,4 butane
. bis(triméthylammonium)-1,3 butane

Parmi les cations répondant à la formule IV, on peut citer les cations:
bis(triphénylphosphine)iminium
bis(tritolylphosphine)iminium.

De préférence, on met en oeuvre un/ou des chlorure(s) d'onium quaternaire.

On recourt avantageusement à ceux des cations onium répondant à la formule (I) ci-avant dans laquelle :
– A représente du phosphore et,
– $R_1$, $R_2$, $R_3$ et $R_4$ sont identiques ou différents et représentent un radical alkyle linéaire ou ramifié contenant de 1 à 8 atomes de carbone, un radical phényle ou méthyl-4 phényle.

On utilise de préférence un chlorure de tétralkylphosphonium.

Le chlorure de tétrabutylphosphonium disponible et particulièrement efficace est plus spécialement recommandé.

Il a été constaté que l'effet bénéfique apporté par la présence dans le milieu de carbonylation d'un sel d'onium quaternaire correspondant à la définition donnée ci-dessus est sensible à partir d'un rapport molaire cation onium/palladium de 0,5 ; notamment un effet particulièrement intéressant a été constaté lorsque ledit rapport est compris entre 1 et 50, un rapport plus élevé, n'étant toutefois pas nocif pour la réaction.

Pour une bonne réalisation du procédé de carbonylation il sera préférable pour le choix du rapport cation onium/palladium de tenir compte de la concentration en palladium du milieu et notamment du rapport molaire butène disubstitué/palladium ; ainsi plus le rapport butène disubstitué/palladium est élevé, plus il y a intérêt à utiliser un rapport cation onium/palladium élevé.

La réaction pourra être généralement conduite en phase liquide à une température comprise entre 50 et 150°C, de préférence entre 80 et 130°C, sous une pression d'oxyde de carbone comprise entre 20 et 250 bar, de préférence entre 90 et 180 bar. Des gaz inertes, tels que l'azote, l'argon ou le gaz carbonique, peuvent être présents à côté de l'oxyde de carbone.

Bien entendu la réaction peut être conduite en présence de solvants ou diluants exogènes au milieu réactionnel tels les hydrocarbures aromatiques, les cétones, les nitriles, les esters ou les amides d'acides carboxyliques.

En fin de réaction ou du temps de réaction voulu, on récupère le diester recherché par tout moyen approprié, par exemple par distillation.

Les exemples ci-après illustrent l'invention ; les rendements sont exprimés par rapport au diacétoxy butène chargé.

EXEMPLE 1 :

Dans un autoclave en Hastelloy B2, on introduit :
30 cm3 (740 mmol) de méthanol, 23 mmol de diacétoxy-1,4 butène-2, 1,25 mmol de $PdCl_2$ et 5,0 g (17 mmol) de chlorure de tétrabutylphosphonium. Le mélange est maintenu pendant 21 heures, avec agitation, sous une pression constante de 165 bar, à 100°C, avec alimentation de monoxyde de carbone pur.

Une analyse par chromatographie en phase gazeuse, par technique quantitative d'étalonnage interne, permet de constater :
– un taux de transformation complet du diacétoxybutène
– un rendement de 65 % en hexène-3 dioate de diméthyle

EXEMPLE 2 :

On opère de la même façon que dans l'exemple 1, mais en réduisant la pression totale à 100 bar et la durée à 6 h. On transforme 95 % du substrat, avec un rendement en hexène-3 dioate de méthyle égal à 43 %.

EXEMPLE 3 :

On opère de la même façon que dans l'exemple 2, mais on remplace le méthanol par un mélange de 10 cm3 de méthanol et 20 cm3 d'acétonitrile. En 6 heures, on transforme 98 % du diacétoxybutène, avec un rendement de 84 % en hexène-3 dioate de méthyle.

EXEMPLE 4 :

Du diacétoxy-1,4 butène-2 est isomérisé en diacétoxy-3,4 butène par chauffage à 100°C, sous atmosphère d'azote en ampoule de verre, en présence de $H_2PtCl_6$ (environ 3 % pondéral).

Le mélange d'isomères (linéaire/branché 2/1), après élimination du platine sur kieselguhr, est soumis à une carbonylation dans les conditions de l'exemple 1. On constate que les deux isomères du diacétoxybutène sont transformés en diester linéaire.

EXEMPLE 5 :

On opère de la même façon que dans l'exemple 3, mais on remplace l'acétonitrile par le même volume de méthylisobutylcétone.

En 6 h, on transforme 92,5 % du diactétoxybutène avec un rendement de 80 % en hexène-3 dioate de méthyle.

EXEMPLE 6 :

On opère de la même façon que dans l'exemple 3 mais en utilisant 10 $cm^3$ de nonanol-1 au lieu du méthanol. On observe la formation d'hexène-3 dioate de dinonyle et d'acétoxypentènoate de nonyle.

EXEMPLE 7 :

On opère de la même façon que dans l'exemple 3 mais on remplace l'acétonitrile par le même volume de toluène.

En 6 h, on transforme 91 % du diacétoxybutène avec un rendement de 82 % en hexène-3 dioate de méthyle.

EXEMPLE 8 :

On reproduit l'exemple 7 à 60°C.

En 6 h, on transforme 60 % du diacétoxybutène avec un rendement de 55 % en hexène-3 dioate de méthyle.

EXEMPLE 9 :

On reproduit l'exemple 7 à 140°C.

En 6 h, on transforme 92 % du diacétoxybutène avec un rendement de 86 % en hexène-3 dioate de méthyle.

EXEMPLE 10 :

On reproduit l'exemple 7 en n'utilisant qu'une mmol de chlorure de tétrabutylphosphonium.

En 6 h, on transforme 89 % du diacétoxybutène avec un rendement de 13 % en hexène-3 dioate de méthyle.

ESSAI TEMOIN a :

On reproduit l'exemple 7 en omettant le chlorure de tétrabutylphosphonium.

En 6 h, on transforme 85 % du diacétoxybutène avec un rendement de 5 % en hexène-3 dioate de méthyle.

EXEMPLE 11 :

On reproduit l'exemple 7 en remplaçant le chlorure de tétrabutylphosphonium par 17 mmol de chlorure de tétraméthylammonium.

En 6 h, on transforme 70 % du diacétoxybutène avec un rendement de 70 % en hexène-3 dioate de méthyle.

EXEMPLE 12 :

On reproduit l'exemple 11 en remplaçant le chlorure de tétrabutylammonium par le chlorure de tétraméthylphosphonium.

En 6 h, on transforme 70 % du diacétoxybutène avec un rendement de 51 % en hexène-3 dioate de méthyle.

EXEMPLE 13 :

Dans l'autoclave et selon le mode opératoire décrits à l'exemple 1, on réalise un essai sur une charge renfermant :

10 cm3 de méthanol
20 cm3 de toluène
23 mmol de diacétoxy-1,4-butène-2
17 mmol de chlorure de tétrabutylphosphonium, et
1 mmol de palladium introduit sous forme de palladium déposé sur du noir de carbone (à 5 % de palladium).

En 6 h, à 100°C, sous une pression constante de 100 bar, avec alimentation de monoxyde de carbone pur on transforme 98 % du diacétoxybutène avec un rendement de 58 % en hexène-3 dioate de méthyle.

EXEMPLE 14 :

On opère de la même façon que dans l'exemple 3 mais on remplace l'acétonitrile par le même volume d'acétate d'éthyle et le méthanol par le même volume d'éthanol.

En 6 heures, on transforme 87 % du diacétoxybutène avec un rendement de 83 % en héxène-3 dioate.

## Revendications

1. Procédé de préparation de diesters de l'acide hexène-3 dioïque par réaction du monoxyde de carbone et d'un alcool sur au moins un butène disubstitué par des groupes acyloxy en présence d'un catalyseur à base de palladium caractérisé en ce que la réaction est conduite également en présence d'un halogénure d'onium quaternaire d'un élément du groupe VB choisi parmi l'azote et le phosphore, ledit élément étant tétracoordonné à des atomes de carbone, l'azote pouvant être coordonné à deux atomes de phosphore pentavalents, l'anion halogénure étant choisi parmi le chlorure et le bromure.

2. Procédé selon la revendication 1, caractérisé en ce que l'halogénure d'onium quaternaire d'un élément du groupe VB choisi parmi l'azote et le phosphore présente un cation onium quaternaire répondant à l'une des formules (I) à (IV) suivantes :

I)

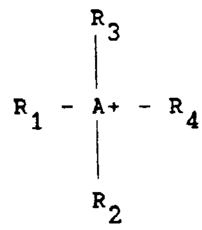

II)

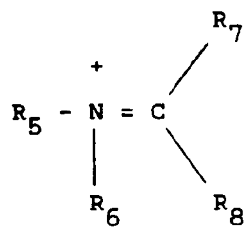

III)

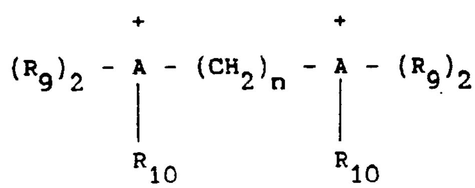

IV)

$$(R_{11})_3 - P = N^+ = P - (R_{11})_3$$

– A représente de l'azote ou du phosphore

– $R_1$, $R_2$,$R_3$, $R_4$ sont identiques ou différents et réprésentent :

. un radical alkyle linéaire ou ramifié contenant de 1 à 16 atomes de carbone, éventuellement substitué par un groupe phényle, hydroxy, halogéno, nitro, alcoxy ou alcoxycarbonyle ;

. un radical alcényle linéaire ou ramifié contenant de 2 à 12 atomes de carbone, de préférence de 4 à 8 atomes de carbone,

. un radical aryle contenant de 6 à 10 atomes de carbone, éventuellement substitué par un ou des radicaux alkyles contenant de 1 à 4 atomes de carbone, alcoxy, alcoxycarbonyle ou halogéno ;

. deux desdits radicaux $R_1$ à $R_4$ pouvant former ensemble un radical alkylène, alcénylène ou alcadiènylène, linéaire ou ramifié et contenant de 3 à 6 atomes de carbone ;

– $R_5$, $R_6$, $R_7$, $R_8$ sont identiques ou différents et représentent :

. un radical alkyle linéaire ou ramifié contenant de 1 à 4 atomes de carbone ;

. les radicaux $R_7$ et $R_8$ pouvant former ensemble un radical alkylène, contenant de 3 à 6 atomes de carbone ;

. les radicaux $R_8$ et $R_7$ ou $R_8$ et $R_8$ pouvant former ensemble un radical alkylène, alcénylène ou alcadiènylène, contenant 4 atomes de carbone et constituant avec N un hétérocycle azoté ;

– $R_9$ représente un radical alkyle linéaire ou ramifié contenant de 1 à 4 atomes de carbone ou un radical phényle ;

– $R_{10}$ représente :

. un radical alkyle linéaire ou ramifié contenant de 1 à 4 atomes de carbone, semblable ou différent de $R_9$ .

. un radical alcényle linéaire ou ramifié, contenant de 2 à 12 atomes de carbone, de préférence de 4 à 8 atomes de carbone ;

– n représente un nombre entier supérieur ou égal à 1 et inférieur ou égal à 10 et de préférence inférieur ou égal à 6 ;

– $R_{11}$ représente un radical aryle contenant de 6 à 10 atomes de carbone, éventuellement substitué par

un ou des groupes alkyle contenant de 1 à 4 atomes de carbone, alcoxy, alcoxycarbonyle ou halogéno.

3. Procédé selon la revendication 1 ou 2, caractérisé en ce que l'halogénure d'onium quaternaire est un chlorure.

4. Procédé selon la revendication 2 ou 3, caractérisé en ce que le cation d'onium quaternaire répond à la formule (I) donnée dans la revendication 2, dans laquelle :

– A représente du phosphore et,

– $R_1$, $R_2$, $R_3$ et $R_4$ sont identiques ou différents et représentent un radical alkyle linéaire ou ramifié contenant de 1 à 8 atomes de carbone, un radical phényle ou méthyl-4 phényle.

5. Procédé selon l'une quelconque des revendications précédentes, caractérisé en ce que l'halogénure d'onium quaternaire est le chlorure de tétrabutylphosphonium.

6. Procédé selon l'une quelconque des revendications précédentes, caractérisé en ce que le rapport molaire du cation ionium au palladium est compris entre 1 et 50.

7. Procédé selon l'une quelconque des revendications précédentes, caractérisé en ce que le rapport molaire du butène disubstitué au palladium est compris entre 2 et 200 et, de préférence, entre 10 et 50.

8. Procédé selon l'une quelconque des revendications précédentes, caractérisé en ce que le rapport molaire de l'alcool au butène disubstitué est compris entre 1 et 100 et, de préférence, entre 1 et 50.

9. Procédé selon l'une quelconque des revendications précédentes, caractérisé en ce que la température de réaction est comprise entre 50 et 150°C et, de préférence, entre 80 et 130°C.

10. Procédé selon l'une quelconque des revendications précédentes, caractérisé en ce que la pression est comprise entre 20 et 250 bar et, de préférence, entre 90 et 180 bar.

11. Procédé selon l'une quelconque des revendications précédentes, caractérisé en ce que le butène disubstitué est choisi parmi le diacétoxy-1,4 butène-2 et le diacétoxy-3,4 butène-1 et leurs mélanges.

## Patentansprüche

1. Verfahren zur Herstellung von Diestern der 3-Hexendisäure durch Umsetzen von Kohlenmonoxid und einem Alkohol mit mindestens einem durch Acyloxygruppen disubstituierten Buten in Gegenwart eines Palladiumkatalysators, dadurch gekennzeichnet, daß die Umsetzung auch in Gegenwart eines quaternären Oniumhalogenids eines Elementes der Gruppe VB, ausgewählt unter Stickstoff und Phosphor, durchgeführt wird, wobei das Element mit Kohlenstoffatomen tetracoordiniert ist, der Stickstoff mit zwei Atomen von fünfwertigem Phosphor coordiniert sein kann und das Halogenanion unter Chlor und Brom ausgewählt ist.

2. Verfahren nach Anspruch 1, dadurch gekennzeichnet, daß das quaternäre Oniumhalogenid eines Elementes der Gruppe VB, ausgewählt unter Stickstoff und Phosphor, ein quaternäres Onium-kation aufweist, das einer der folgenden Formeln (I) bis (IV) entspricht:

I)

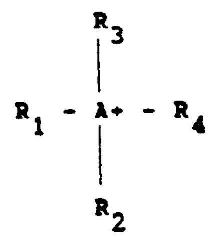

II)

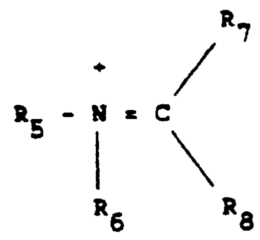

III)

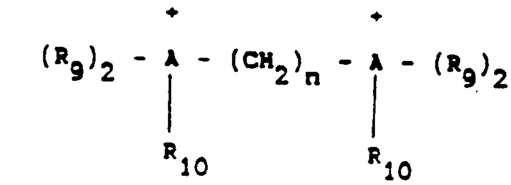

IV)

$$(R_{11})_3 - P = N^+ = P - (R_{11})_3$$

in denen

– A Stickstoff oder phosphor bedeutet,

– $R_1$, $R_2$, $R_3$ und $R_4$ gleich oder verschieden sind und jeweils für:

. einen linearen oder verzweigten Alkylrest mit 1 bis 16 Kohlenstoffatomen, gegebenenfalls substituiert durch eine phenyl-, Hydroxy-, Halogen-, Nitro-, Alkoky- oder Alkoxycarbonylgruppe;

. einen linearen oder verzweigten Alkenylrest, der 2 bis 12 Kohlenstoffatome, vorzugsweise 4 bis 8 Kohlenstoffatome, enthält;

. einen Arylrest, der 6 bis 10 Kohlenstoffatome enthält, gegebenenfalls substituiert mit einem oder mehreren Alkylresten, die 1 bis 4 Kohlenstoffatome enthalten, Alkoxy, Alkoxycarbonyl oder Halogen, stehen;

. zwei der Substituenten $R_1$ bis $R_4$ zusammen einen linearen oder verzweigten Alkylen-, Alkenylen- oder Alkadienylenrest mit 3 bis 6 Kohlenstoffatomen bilden können;

– $R_5$, $R_6$, $R_7$ und $R_8$ gleich oder verschieden sind und jeweils für:

. einen linearen oder verzweigten Alkylrest mit 1 bis 4 Kohlenstoffatomen stehen;

. die Substituenten $R_7$ und $R_8$ zusammen einen Alkylenrest mit 3 bis 6 Kohlenstoffatomen bilden können;

. die Substituenten $R_8$ und $R_7$ oder $R_6$ oder $R_8$ zusammen einen Alkylen-, Alkenylen- oder Alkadienylenrest mit 4 Kohlenstoffatomen bilden können, der mit N zusammen einen stich.stoffhaltigen Heterozyklus bildet;

– $R_9$ für einen linearen oder verzweigten Alkylrest mit 1 bis 4 Kohlenstoffatomen oder für einen Phenylrest steht;

– $R_{10}$ für:

. einen linearen oder verzweigten Alkylrest mit 1 bis 4 Kohlenstoffatomen gleich oder verschieden mit/von $R_9$ oder

. einen linearen oder verzweigten Alkenylrest mit 2 bis 12 Kohlenstoffatomen, vorzugsweise mit 4 bis 8 Kohlenstoffatomen, steht;

– n eine ganze Zahl $\geqq 1$ und $\leqq 10$, vorzugsweise $\leqq 6$ ist;

– $R_{11}$ einen Arylrest, der 6 bis 10 Kohlenstoffatome enthält, gegebenenfalls substituiert mit einer oder mehreren Alkylgruppen, die 1 bis 4 Kohlenstoffatome enthalten, Alkoxy, Alkoxycarbonyl oder Halogen, bedeutet.

3. verfahren nach Anspruch 1 oder 2, dadurch gekennzeichnet, daß das quaternäre Oniumhalogenid ein Chlorid ist.

4. Verfahren nach Anspruch 2 oder 3, dadurch gekennzeichnet, daß das quaternäre Oniumkation der Formel (I) in Anspruch 2 entspricht, in der:

– A phosphor bedeutet und

– $R_1$, $R_2$, $R_3$ und $R_4$ gleich oder verschieden sind und jeweils einen linearen oder verzweigten Alkylrest mit 1 bis 8 Kohlenstoffatomen, einen phenyl- oder 4-Methylphenylrest bedeuten.

5. verfahren nach einem der vorangehenden Ansprüche, dadurch gekennzeichnet, daß das quaternäre Oniumhalogenid Tetrabutylphosphoniumchlorid ist.

6. verfahren nach einem der vorangehenden Ansprüche, dadurch gekennzeichnet, daß das Molverhältnis von Oniumkation zu palladium 1 bis 50 ausmacht.

7. verfahren nach einem der vorangehenden Ansprüche, dadurch gekennzeichnet, daß das Molverhältnis vor. disubstituiertem Buten zu palladium 2 bis 200 und vorzugsweise 10 bis 50 ausmacht.

8. verfahren nach einem der vorangehenden Ansprüche, dadurch gekennzeichnet, daß das Molverhältnis von Alkohol zu disubstituiertem Buten 1 bis 100 und vorzugsweise 1 bis 50 ausmacht.

9. verfahren nach eine der vorangehenden Ansprüche, dadurch gekennzeichnet, daß die Reaktionstemperatur im Bereich von 50 bis 150°C, vorzugsweise von 80 bis 130°C liegt.

10. verfahren nach einem der vorangehenden Ansprüche, dadurch gekennzeichnet, daß der Druck im Bereich von 20 bis 250 bar und vorzugsweise von 90 bis 180 bar liegt.

11. verfahren nach einem der vorangehenden Ansprüche, dadurch gekennzeichnet, daß das disubstituierte Buten unter 1,4-Diacetoxy-2-buten ur.d 3,4-Diacetoxy-1-buten sowie deren Gemischer. ausgewählt wird.

## Claims

1. Process for the preparation of diesters of 3-hexenedioic acid by reaction of carbon monoxide and of an alcohol with at least one butene disubstituted by acyloxy groups, in the presence of a catalyst based on palladium, characterised in that the reaction is also conducted in the presence of a quaternary onium halide of an element of group VB chosen from nitrogen and phosphorus, the said element being tetracoordinated to carbon atoms, it being possible for the nitrogen to be coordinated to two pentavalent phosphorus atom, the halide anion being chosen from chloride and bromide.

2. Process according to Claim 1, characterised in that the quaternary onium halide of an element of group VB chosen from nitrogen and phosphorus has a quaternary onium cation corresponding to one of the following formulae (I) to (IV):

I)

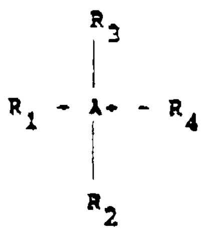

II)

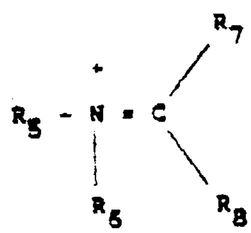

III)

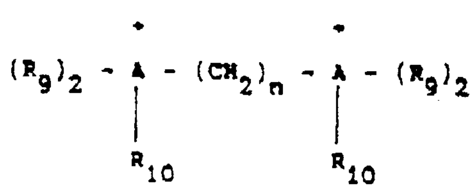

IV)

$$(R_{11})_3 - P = N^+ = P - (R_{11})_3$$

– A denotes nitrogen or phosphorus

– $R_1$, $R_2$, $R_3$ and $R_4$ are identical or different and denote:

. a linear or branched alkyl radical containing from 1 to 16 carbon atoms, optionnally substituted by a phenyl, hydroxyl, halo, nitro, alkoxy, or alkoxycarbonyl, group ;

. a linear or branched alkenyl radical containing from 2 to 12 carbon atoms, preferably from 4 to 8 carbon atoms,

. an aryl radical containing from 6 to 10 carbon atoms, optionnaly substituted by one or more alkyl radicals containing from 1 to 4 carbon atoms, or alkoxy, alkoxycarbonyl or halo radicals;

. it being possible for two of the said radicals $R_1$ to $R_4$ together to form a linear or branched alkylene, alkenylene or alkadienylene radical containing from 3 to 6 carbon atoms;

– $R_5$, $R_6$, $R_7$ and $R_8$ are identical or different and denote:

. a linear or branched alkyl radical containing from 1 to 4 carbon atoms;

. it being possible for the radicals $R_7$ and $R_8$ together to form an alkylene radical containing from 3 to 6 carbon atoms;

. it being possible for the radicals $R_6$ and $R_7$ or $R_6$ and $R_8$ together to form an alkylene, alkenylene or alkadienylene radical containing 4 carbon atoms and forming a nitrogenous heterocyclic ring with N;

– $R_9$ denotes a lined or branched alkyl radical containing from 1 to 4 carbon atoms or a phenyl radical;

– $R_{10}$ denotes:

. a linear or branched alkyl radical containing from 1 to 4 carbon atoms, similar to or different from $R_9$;

. a linear or branched alkenyl radical containing from 2 to 12 carbon atoms, preferably from 4 to 8 carbon atoms;

– n denotes an integer higher that or equal to 1 or lower than or equal to 10 and preferably lower than or equal to 6;

– $R_{11}$ denotes an aryl radical containing from 6 to 10 carbon atoms, optionally substituted by one or more alkyl groups containing from 1 to 4 carbon atoms, or alkoxy, alkoxycarbonyl or halo groups.

3. Process according to Claim 1 or 2, characterized in that the quaternary onium halide is a chloride.

4. Process according to Claim 2 or 3, characterised in that the quaternary onium cation corresponds to the formula (I) given in Claim 2, in which:

– A denotes phosphorus, and

– $R_1$, $R_2$, $R_3$, and $R_4$ are identical or different and denote a linear or branched alkyl radical containing from 1 to 8 carbon atoms, or a phenyl or 4-methylphenyl radical.

5. Process according to any one of the preceding claims, characterised in that the quaternary onium halide is tetrabutylphosphonium chloride.

6. Process according to any one of the preceding claims, characterised in that the molar ratio of the onium cation to palladian is between 1 and 50.

7. Process according to any one of the preceding claims, characterised in that the molar ratio of the disubstituted butene to palladium is between 2 and 200, and preferably between 10 and 50.

8. Process according to any one of the preceding claims, characterized in that the molar ratio of the alcohol to the disubstituted butene is between 1 and 100, and preferably between 1 and 50.

9. Process according to any one of the preceding claims, characterised in that the reaction temperature is between 50 and 150°C, and preferably between 80 and 130°C.

10. Process according to any one of the preceding claims, characterised in that the pressure is between 20 and 250 bar, and preferably between 90 and 180 bar.

11. Process according to any one of the preceding claims, characterised in that the disubstituted butene is chosen from 1,4-diacetoxy-2-butene and 3,4-diacetoxy-1-butene and mixtures thereof.